Europäisches Patentamt

European Patent Office

Office européen des brevets

(19)

(11) Veröffentlichungsnummer: **0 352 325**
**A1**

(12) **EUROPÄISCHE PATENTANMELDUNG**
veröffentlicht nach Art. 158 Abs. 3
EPÜ

(21) Anmeldenummer: 88903994.7

(22) Anmeldetag: 12.01.88

(86) Internationale Anmeldenummer:
**PCT/SU88/00010**

(87) Internationale Veröffentlichungsnummer:
**WO 89/06551 (27.07.89 89/16)**

(51) Int. Cl.⁴: **A61M 25/00**

(43) Veröffentlichungstag der Anmeldung:
**31.01.90 Patentblatt 90/05**

(84) Benannte Vertragsstaaten:
**CH DE FR GB IT LI SE**

(71) Anmelder: **KIEVSKY
NAUCHNO-ISSLEDOVATELSKY INSTITUT
NEIROKHIRURGII
ul. Manuilskogo, 32
Kiev, 252655(SU)**

(72) Erfinder: **SCHEGLOV, Viktor Ivanovich
ul. Belorusskaya, 15b-51
Kiev, 252119(SU)**
Erfinder: **SAVENKO, Alexandr Grigorievich
ul. Murashko, 5-100
Kiev, 252050(SU)**
Erfinder: **GONCHAROV, Alexandr Ivanovich
ul. Generala Koltsova, 40-39
Kiev, 252146(SU)**
Erfinder: **ANTONENKO, Vyacheslav
Grigorievich
ul. Volgo-Donskaya, 67-4
Kiev, 252099(SU)**

(74) Vertreter: **Görg, Klaus, Dipl.-Ing. et al
Hoffmann, Eitle & Partner Arabellastrasse 4
(Sternhaus)
D-8000 München 81(DE)**

(54) **OKKLUSIONSMITTEL.**

(57) Okklusierungsvorrichtung, die einen abnehmbaren, aufblasbaren, vermittels des Sphinkters (2) mit dem einsetzbaren Katheter (3) gekoppelten Ballon (1) und einen seinen eigenen Katheter (5) aufweisenden, im Inneren des Hauptballons (1) untergebrachten Zusatzballon (4) einschließt, wobei ein der erwähnten Ballons an seiner Oberfläche die durchgehende Perforation (7) besitzt.

FIG 1

## OKKLUSIONSVORRICHTUNG

### Technisches Gebiet

Die vorliegende Erfindung bezieht sich auf das Gebiet Medizin, insbesondere auf Röntgenchirurgie, chirurgische Radiologie, und zwar auf Okklusionsvorrichtungen und kann bei der Behandlung verschiedenartiger Gefäßerkrankungen, der Gefäßaneurysmen großer Abmessungen, unterschiedlicher Form und Konfiguration verwendet werden.

### Zugrundeliegender Stand der Technik

Gegenwärtig stellen die Erkrankungen der Gehirngefäße, des Herzens und anderer Organe die verbreitesten Erkrankungen des Menschens dar. Zu den wirksamen Behandlungsverfahren der Gefäßerkrankungen zählen die chirurgischen Verfahren der Heilbehandlungen. Jedoch bei deren ausreichender Wirksamkeit zeichnen sich die chirurgischen Benandlungsverfahren durch ihre negativen Auswirkungen wie hohe Verletzbarkeit, insbesondere bei den Operationen an Gehirngefäßen, schwere Nachoperationskomplikationen aus. Die zur letzten Zeit an ihrer Entwicklung gewinnende Röntgenchirurgie bringt auch verkleinerte Verletzbarkeit, verminderte Nachoperationskomplikationen mit sich sowie ermöglicht es, die operative Behandlung der Gefäßerkrankungen in solchen Regionen durchzuführen, in welchen die herkömmliche Chirurgie und Mikrochirurgie der Gefäße unmöglich ist. Besondere Schwierigkeiten bei der röntgenchirurgischen Aneurysmenbehandlung bereiten Aneurysmen von großen und riesengroßen Ausdehnungen, falsches Aneurysma und Aneurysma mit breitem Hals. Gegenwärtig wird die Benandlung der erwähnten Aneurysmenarten vermittels der röntgenchirurgischen Verfahren auf eine stationäre Okklusion der das Aneurysma tragenden Gefäße zusammengeführt. Die betreffenden Operationen sind in der Regel mit erheblichen Nachoperationskomplikationen verbunden, die Ischemie der großen Gewebefelder, insbesondere im Gehirn verursacht.

Die Unvollständigkeit der zur Anwendung kommenden Röntgenoperationsinstrumente aber erschwert die Lösung eines der wichtigsten Probleme der Behandlung der Gefäßerkrankungen.

- 2 -

Die Weiterentwicklung der Röntgenchirurgie bedarf daher der Entwicklung von grundsätzlich neuen Okklusionsvorrichtungen zur Durchführung der jeweiligen Operationen.

Bekannt ist eine Okklusionsvorrichtung (Patentschrift GB 2 045 621, A), die aus einem abtrennbaren Aufblaseballon besteht, der mit dem Katheter vermittels eines Übergangsstücks verbunden ist. Dieser Ballon wird durch Verschmelzen des Übergangsstücks von dem Katheter abgetrennt. Das Verschmelzen wird vermittels einer durch die Drähte mit einer Stromquelle verbundenen, elektrischen Spirale zustandegebracht. Die erwähnten Verbindungsdrähte werden im Inneren des Katheters hindurchgezogen, während die Elektrospirale durch eine zusätzliche, elektroisolierende Muffe gegen die Umgebung abgesondert wird.

Durch die betreffende, aufbaumäßige Ausführung des Ballonkatheters wird eine sichere Abtrennung des Ballons von dem Katheter erreicht, der Katheter selbst aber erhält dabei eine übermäßige Starrheit, die dessen Einführung ins Innere des jeweiligen Aneurysmas und in die Abzweiggefäße unmöglich macht. Die mit Hilfe des Ballonkatheters der erwähnten Bauart durchgeführten Operationen sind nur auf die stationäre Okklusion der zuleitenden Gefäße und der ein Aneurysmatragenden Gefäße zusammengeführt. Dadurch treten starke Veränderungen des Blutkreislaufs in dem betreffenden Bereich, blutleere Regionen auf.

Die erwähnten Umstände sind insbesondere bei der Durchführung der röntgenchirurgischen Operationen an den Gehirngefäßen äußerst unerwünscht.

Die obenbeschriebene Vorrichtung ermöglicht in einigen Fällen die Einführung in den Innenraum der Aneurysmen von großen und riesengroßen Ausdehnungen, der Aneurysmen mit dem breiten Hals, trotzdem reicht ein Ballon zu deren Ausschaltung aus dem Blutkreislauf nicht, während die Einführung mehrerer Ballone in den Innenraum des jeweiligen Aneurysmas ohne deren gegenseitige Zusammenkoppelung zustandekommt und die durch den Blutstrom hervorgerufene Wanderung der erwähnten Ballone in dem Innenraum des Aneurysmas zu deren Ausschwemmung aus den

- 3 -

Aneurysmen und demzufolge auch zu der Okklusion der lebenswichtigen Gefäße führt.

Bekannt ist auch ein Ballon-Katheter mit getrennten Ballonen und Verbindungskanälen (DE, 3 048 923 C$_2$). Diese Vorrichtung besitzt zwei Ballone. Ein Ballon, nähmlich Stellungsballon besorgt die Zurückhalterung der Vorrichtung an der erforderlichen Stelle und zweiter Ballon stellt den Okklusionsballon dar. Bei deren Aufblasen nehmen beide Ballone eine Gesamtform unterschiedlicher Gestaltung an. Die getrennten Verbindungskanäle dienen zur getrennten Einführung des jeweiligen Kontrastmittels und der Bestandteile eines schellhärtenden Polymeres. Der betreffende Ballon-Katheter ermöglicht die Einstellung der Polymerisationszeit des schnellhärtenden Polymeres durch Vorliegen der selbsttätigen Verbindungskanäle, Katheter zur Ausfüllung des Innenraums des jeweiligen Ballons, welche eine getrennte Eingabe in den Ballon der Bestandteile des schnellhärtenden Polymeres bei deren jeweils passendem Mengenverhältnis zu verwirklichen gestatten. Der derart ausgeführte Ballon-Katheter wird nach seiner Zweckbestimmung zur Verschließung der Mangel der Kammerscheidewand und der Vorhofscheidewand und infolge seiner verengten Zweckbestimmung nur innerhalb des eingeschränkten Anwendungsbereich ausgenutzt.

Allgemein bekannt ist weiter ein Devonin-Ballon-Katheter (FR, A, 2 383 673). Dieser Devonin-Ballon-Katheter ist in Form eines abnehmbaren, aufblasbaren Ballons mit einer Röntgenkontrastmarke ausgeführt, die einen in der dem Sphinkter gegenüberliegenden Verdickung der Ballonwandung eingesetzten Klipps darstellt. Der Ballonsphinkter ist dabei ins Innere des Ballons eingekrümmt ausgeführt und auf der Außenwandung des Katheters aufgesetzt. Beim Ausschalten aus dem Kreislauf eines Gefäßes, Aneurismas, wird der Ballon durch seine Auffüllung mit einem schnellhärtendem Polymer auseinandergezogen, während der Katheter unter Umstülpung des Ballonssphinkters außerhalb des Ballons entfernt wird. Zur Abnahme (Abtrennung des Ballons) von dem Katheter wird ein auf den Arbeitskatheter (Haupt-

- 4 -

katheter) aufgeschobener Abschiebekatheter eines größeren Durchmessers ausgenutzt. Beide Katheter werden in entgegengesetzten Richtungen verschoben, und zwar der Hauptkatheter wird herausgezogen und der Abschiebekatheter in Richtung des Ballons zu hineingegeben. Auf diese Weise werden beide Katheter nach der Abschiebung des Ballons von dem Arbeitskatheter aus dem Gefäß herausgenommen. In seinem Aufbau weist der Devchin-Ballon-Katheter eine übermäßige Starrheit auf, die die Einführung des Ballons in die Abzweiggefäße und in die Höhlen der Aneurismen nicht ermöglicht, was die Anwendungsgrenzen des betreffenden Ballon-Katheters herabsetzt, während die mit Hilfe des Devchin-Ballon-Katheters durchgeführten Operationen in der Regel auf eine stationäre Okklusion der zuleitenden Gefäße, der die Aneurysmen tragenden Gefäße eingerichtet werden, wodurch eine weitgehende Veränderung der Blutversorgung und Nachoperationskomplikationen auftreten. Bei den erfolgreichen Versuchen, den Devchin-Ballon-Katheter in die Höhle der großen und riesengrossen Aneurysmen, der Aneurysmen mit dem breiten Hals zu deren Ausschließung aus dem Blutkreislauf einzuführen, müssen in die jeweilige Aneurysmahöhle bis 10 Ballons eingeführt werden, die trotzdem in dieser Höhle des Aneurysmas nicht zurückgehalten werden können und durch den Blutstrom herausgeschwemmt werden. Die aus dem Blutstrom ausgeschalteten Gefäßaneurysmen rezidivieren in mehreren Fällen durch Blutungen.

## Offenbarung der Erfindung

Der vorliegenden Erfindung liegt die Aufgabe zugrunde eine solche Okklusionsvorrichtung zu entwickeln, die es möglich macht, durch deren Ausstattung mit einem Zusatzballon mit hohem Sicherheitsgrad aus dem Blutstrom die Aneurysmen selbst unterschiedlicher Konfiguration und großer Ausdehnungen auszuschalten.

Die gestellte Aufgabe wird dadurch gelöst, daß die Okklusionsvorrichtung, die einen abnehmbaren, aufblasbaren, vermittels Sphinkters mit dem einsetzbaren Katheter gekoppelten Ballon besitzt, erfindungsgemäß mit einem seinen eigenen Katheter aufweisenden, innerhalb des Hauptballons untergebrachten Zusatzballon mit einer röntgenkontrasten Marke versehen ist, wobei ein der betreffenden
Ballons auf seiner Oberfläche eine durchgehende Perforation aufweist.

In diesem Zusammenhang besteht die Möglichkeit, das
Problem des sogenannten "toten" Raums des Katheters zu
lösen, in die Aneurysmahöhle den klebenden, thrombenbildenden Stoff, die Bestandteile eines schnellhärtenden Polymeres getrennt einzuführen und somit die Polymerisationszeit des betreffenden, schnellhärtenden Polymeres in dem
Innenraum des jeweiligen Ballons zu bestimmen und nachzustellen.

Dadurch wird es möglich, vermittels der Röntgenchirurgie die Aneurysmenbehandlung bei den verschiedenen
Konfigurationen und großen Ausdehungen der Aneurismen,
die früher keiner Behandlung ausgesetzt werden konnten,
durchzuführen.

<u>Kurzbeschreibung der Zeichnungen</u>

Nachstehend wird die Erfindung an hand der Beschreibung der konkreten Ausführungsbeispiele und angelegten
Zeichnungen näher erläutert, in denen es zeigt:

Fig. 1 erfindungsgemäße Okklusionsvorrichtung in Gesamtansicht mit der Perforation in dem Außenballon;

Fig. 2 Wirkungsweise der erfindungsgemäßen Okklusionsvorrichtung aus der Fig. 1;

Fig. 3 erfindungsgemäßes Ausschaltungsbeispiel des
aus dem Blutstrom ausgeschalteten Aneurysmas der großen
Ausdehnung nach der Einführung des Ballons und dem Auszug des Katheters;

Fig. 4 erfindungsgemäße Okklusionsvorrichtung in Gesamtansicht mit der Perforation in der Wandung des inneren Ballons;

Fig. 5 Wirkungsweise der erfindungsgemaßen Okklusionsvorrichtung aus der Fig. 4;

Fig. 6 erfindungsgemäßes Beispiel der getrennten Einführung der Bestandteile eines schnellhärtenden Polymeres in die Aneurysmahöhle (schematische Darstellung).

Bester Weg zur Ausführung der Erfindung

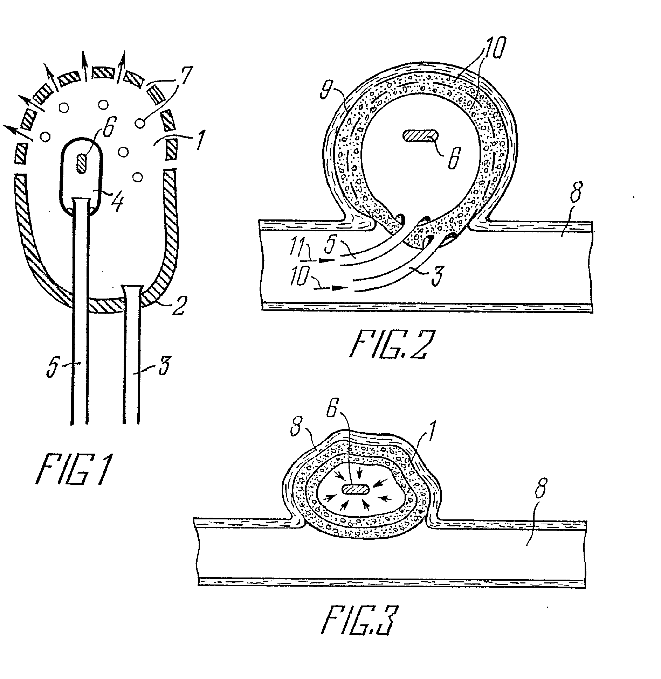

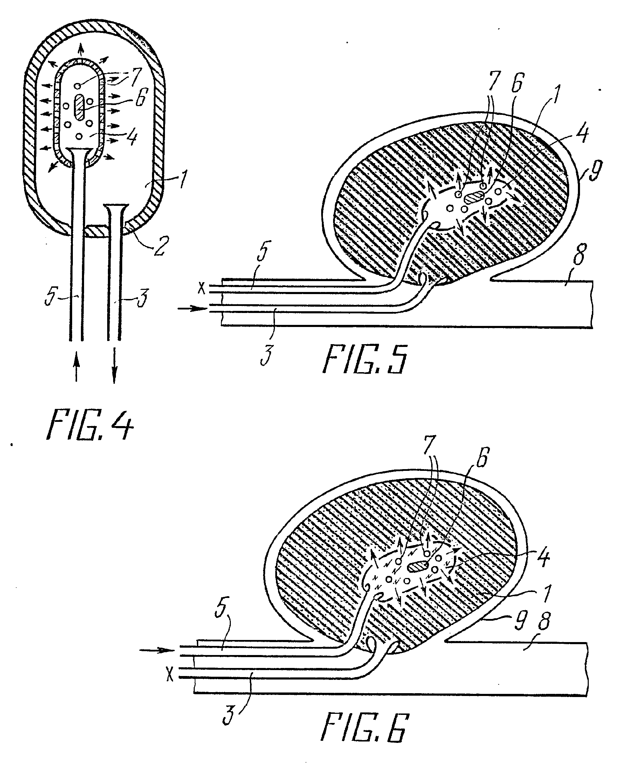

Die erfindungsgemäße Okklusionsvorrichtung enthält einen aufblasbaren Außenballon 1 (Fig. 1, 4), der mit Hilfe von Sphinkter 2 mit dem einsetzbaren Katheter 3 verbunden ist. Erfindungsgemäß ist die Vorrichtung mit einem inneren Zusatzballon 4 versehen, der seinen eigenen Katheter besitzt, wobei in dem Innenraum des betreffenden Zusatzballons 4 eine röntgenkontraste Marke 6 untergebracht ist, während ein der erwähnten Ballons (1) die durchgehende Perforation 7 aufweist.

Vor Beginn der Durchführung der röntgenchirurgischen Operation werden die Behandlungsart und die Gestaltung des verwendbaren Ballons gewählt, und zwar das Vorliegen der Perforation in dem inneren Zusatz- oder in dem Außenballon.

Der Außenballon 1 mit dem in diesem untergebrachten Zusatzballon 4 und der röntgenkontrasten Marke 6 wird vermittels der Katheter 3 und 5 durch die Punktionsnadel in das arterielle Gefäß 8 eingeführt und in die Höhle des Aneurysmas 9 weitergeschoben (Fig. 2,5). Die Röntgenkontrastmarke 6 gibt dabei die Möglichkeit, den Stellungslage beider Ballons 1 und 4 innerhalb der Höhle des Aneurysmas 9 unter Kontrolle zu halten. Je nach der gewählten Operationsart führt man dem Ballon 1 über den Katheter 3 einen klebenden Füllstoff 10, den Hauptbestandteil 11 des schnellhärtenden Polymeres zu. Dann werden über den Katheter 5 (Fig. 6) die Restbestandteile der Auffüllung der Ballons zur Beendigung der Operation eingeführt. Fig. 3 gibt die Endphase der Operation wieder.

Bei der Diagnostik eines arteriellen Aneurysmas von großen Abmessungen mit einem breiten bzw. normalen Hals wird die Okklusionsvorrichtung mit der durchgehenden Perforation des Außenballons (Fig. 1) zur röntgenchirurgischen Operation gewählt. Nach der durchgeführten medikamentösen Prämedikation wird die Arterie bei dem Kranke punktiert. Unter Röntgenfernsehekontrolle werden die durch die Punktionsnadel hineingeschobenen Ballone 1, 4 und Katheter 3, 5 innerhalb der Arterie 8 an die Mündung des Aneurysmas 9 zugeführt. Hiernach wird der Außenballon 1 samt dem Zusatzballon 4 in die Höhle des Aneurysmas 9 hineingeschoben. Durch den Katheter 5 wird ein röntgenkontrastes Mittel unter gleichzeitiger Röntgenfernsehekontrolle bis zur Obturation der Höhle des Aneurysmas 9 eingeführt.

Hiernach wird das Röntgenkontrastmittel in dem Katheter 5 des Zusatzballons 4 herausgezogen und dessen Menge ermittelt. Nun kann durch den Katheter 5 ein schnellhärtendes Polymer, beispielsweise Silikon in den Zusatzballon 4 bis zur Obturation des Halses des Aneurysmas 9 eingeführt. Zu dieser Zeit wird auch der thrombenbildende Klebestoff 10 über den Katheter 3 in den Innenraum des Außenballons 1 mit Hilfe einer Spritze eingeführt, das durch die durchgehende Perforation 7 in die Höhle des Aneurysmas 9 hineindringt, indem dadurch die Wandungen beider Ballone 1, 4 und des Aneurysmas 9 (Fig. 2) zuverlässig zusammengeklebt werden. Nach der Polymerisation des schnellhärtenden Polymeres werden die Katheter 3,5 von den Ballons 1,4 vermittels eines der bekannten Verfahren abgetrennt. Bei der weiteren Polymerisation nimmt Silikon 12 an seinem Rauminhalt ab und die verklebten Wandungen der Ballons 1,4 und des Aneurysmas 9 werden mitgerissen, wodurch auch die Verkleinerung des Rauminhaltes des früheren Aneurysmas 9 (Fig. 3) zustandekommt.

Nach der Diagnostizierung des Aneurysmas mittlerer Größe einer verwickelten Gestaltung aber mit einem breiten Hals wird die Okklusionsvorrichtung mit der durchgehenden Perforation in dem inneren Zusatzballon 4 (Fig. 4) zur Durchführung der röntgenchirurgischen Operation gewählt. Unter Röntgenfernsehekontrolle werden die Ballons 1, 4 (Fig. 2) mit den Kathetern 3,5 nach dem oberbeschriebenen Verfahren unter Ausnutzung der Besonderheiten der Hämodynamik des Blutes durch die Arterien an die Mündung des Aneurysmas und hiernach auch in seine Höhle eingeführt. Über den Katheter 3 wird der Außenballon 1 mit dem Hauptbestandteil des schnellhärtenden Polymeres bis zur ganzen Obturation der Höhle des Aneurysmas 9 ausgefüllt, was durch Angioskopie gut kontrolliert wird. In den Innenraum

- 8 -

des Zusatzballons 4 wird ein Katalysator in seiner vorbestimmten Menge über den Katheter 5 vermittels der Spritze eingeführt. Dieser Katalysator tritt durch die durchgehende Perforation 7 des Zusatzballons 4 in den Innenraum des Außenballons 1 hinein und bewirkt dort die schnellverlaufende Polymerisation des Hauptbestandteils, des schnellerstarrenden Polymeres 12 (Fig. 6). Nach der erfolgten Polymerisation des schnellhärtenden Polymeres werden auf eine der bekannten Weisen die Katheter 3, 5 von den Ballonen 1, 4 abgetrennt und dann wird die serienmäßige Kontrollangiographie durchgeführt.

## Gewerbliche Verwendbarkeit

Die erfindungsgemäße Okklusionsvorrichtung ist vorzugsweise zum Ausschalten der mittleren, großen und riesengroßen Aneurysmen der großen Leitungsgefäße im Gehirn, Hals, Organen usw. aus dem Blutstrom vorausbestimmt. Die erwähnte Ausschaltung der erwähnten Aneurysmen des menschlichen Organismus aus dem Blutstrom bereitet bis zur letzten Zeit bei der Benutzung der herkömmlichen Ballonkatheter wesentliche Schwierigkeiten. Die erfindungsgemäßen Okklusionsvorrichtungen ermöglichen es, die klebenden, thrombenbildenden Stoffe sowie die Mischungen eines schnellhärtenden Polymeres in die Innenräume der Aneurysmen und Ballons getrennt einzuführen. Dadurch entsteht die Möglichkeit, mit einem hohen Sicherheitsgrad ohne Rezidivierungen die Behandlung der erwähnten Aneurysmen auf dem röntgenchirurgischen Wege durchzuführen, an denen bisher solche Behandlung unter Erhaltung des physiologischen Blutstroms keinerlei erfolgreich durchgeführt werden konnte. Durch die Besonderheiten der aufbaumäßigen Ausführung der erfindungsgemäßen Okklusierungsvorrichtungen wird eine verlangsamte Verminderung des Rauminhaltes der früheren Aneurysmen ermöglicht, was bei der Behandlung der Aneurysmen der Gehirngefäße von besonderer Bedeutung und wertvoll ist, da dabei der Preßdruck der großen Aneurysmen an den Gehirnumgebungsstrukturen abnimmt.

Darüber hinaus bringen die erfindungsgemäßen Okklusionsvorrichtungen eine zuverlässige Vorbeugung der

eventuellen Wiederholung der Aneurysmarupturen und Blutung mit sich.

PATENTANSPRUCH:

Okklusierungsvorrichtung, die einen vermittels des Sphinkters (2) mit dem einsetzbaren Katheter (3) verbundenen, aufblasbaren Ballon (1) einschließt, d a d u r c h g e k e n n z e i c h n e t, daß sie mit einem inneren Zusatzballon (4) mit der röntgenkontrasten Marke (6) versehen ist, der seinen eigenen Katheter (5) aufweist und im Inneren des Hauptballons (1) untergebracht ist, wobei ein dieser Ballons an seiner Oberfläche die durchgehende Perforation (7) besitzt.

FIG 1

FIG.2

FIG.3

FIG.4

FIG.5

FIG.6

# INTERNATIONAL SEARCH REPORT

International Application No PCT/SU 88/00010

## I. CLASSIFICATION OF SUBJECT MATTER (if several classification symbols apply, indicate all) 6

According to International Patent Classification (IPC) or to both National Classification and IPC

IPC$^4$: A 61 M 25/00

## II. FIELDS SEARCHED

| Minimum Documentation Searched 7 | |
|---|---|
| Classification System | Classification Symbols |
| IPC$^4$: | A 61 M 25/00 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | US, A, 4517979, (Cordis Corporation), 21 May 1985, see column 2 lines 55-65, column 4 lines 5-20, fig. 5 | 1 |
| A | SU, A1, 787037, (Nauchno-issledovatelsky ordena Trudovogo Krasnogo Znameni institut neirokhirurgii im. N.N. Burdenko AMN SSSR), 15 December 1980, see column 1 lines 20-25, column 2 lines 10-20, column 3 lines 5-10 | 1 |
| A | SU, A1, 787039, (Institut neirokhirurgii im. akad. N.N. Burdenko AMN SSSR), 15 December 1980, see column 2 lines 20-30, column 3, lines 5-20 | 1 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 20 July 1988 (20.07.88) | 5 October 1988 (05.10.88) |
| International Searching Authority | Signature of Authorized Officer |
| ISA/SU | |

Form PCT/ISA/210 (second sheet) (January 1985)